(19)

Europäisches
Patentamt
European
Patent Office
Office européen
des brevets

(11)  **EP 2 850 993 A1**

(12)  **EUROPEAN PATENT APPLICATION**
published in accordance with Art. 153(4) EPC

(43) Date of publication:
**25.03.2015  Bulletin 2015/13**

(51) Int Cl.:
***A61B 1/00*** (2006.01)    ***A61B 1/04*** (2006.01)
***G02B 23/24*** (2006.01)    ***H04N 7/18*** (2006.01)

(21) Application number: **13791645.8**

(22) Date of filing: **23.04.2013**

(86) International application number:
**PCT/JP2013/061869**

(87) International publication number:
**WO 2013/172156 (21.11.2013 Gazette 2013/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:  **18.05.2012  JP 2012114338**

(71) Applicant: **HOYA Corporation
Tokyo 161-8525 (JP)**

(72) Inventor: **CHIBA, Toru
Tokyo 161-8525 (JP)**

(74) Representative: **Schaumburg, Thoenes, Thurn,
Landskron, Eckert
Patentanwälte
Postfach 86 07 48
81634 München (DE)**

(54)  **ELECTRONIC ENDOSCOPE DEVICE**

(57)   The electronic endoscope device is provided with: a spectral image capturing means for capturing a spectral image of a predetermined wavelength range in a body cavity and obtaining spectral image data; a spectrum resolving means whereby the spectral data of each of the pixels included in the spectral image data is resolved into a plurality of predetermined component spectra by performing regression analysis; a spectrum compositing means for recomposing the predetermined plurality of component spectra, removing at least one of the plurality of component spectra, and thereby generating composited image data; and a display means for performing screen display on the basis of the composite image data.

FIG. 5

EP 2 850 993 A1

**Description**

**TECHNICAL FIELD**

The present invention relates to an electronic endoscope device capable of emitting light in different wavelengths at a living tissue and capturing a spectral image.

**BACKGROUND ART**

Recently, as described, for example, in Japanese Patent Provisional Publication No. JP2007-135989A, an electronic endoscope equipped with a function to capture spectral images has been proposed. With such an electronic endoscope, it may be possible to obtain image information containing spectral property (frequency characteristic of light absorption property) of a living tissue such as a mucous membrane in a digestive organ, which is, for example, a stomach or a rectum. It is known that the spectral property of a living tissue reflects information concerning types or densities of components contained in the vicinity of a surface layer of the subject living tissue. In particular, the spectral property of the living tissue can be obtained by superimposing spectral properties of a plurality of essential components which constitute the living tissue.

A diseased portion in a living tissue may contain a greater amount of substance, which is rarely contained in a healthy portion in the living tissue. Therefore, a spectral property of the living tissue containing the diseased portion may tend to differ from a spectral property of the living tissue containing only the healthy portion. Thus, while the spectral properties of the healthy portion and the diseased portion are different from each other, it may be possible to determine whether or not the living tissue contains any diseased portion by comparing the spectral properties of the healthy portion and the diseased portion.

Meanwhile, wavelength characteristics of scattering coefficients on human skin or mucous membrane have been researched, and it has been reported that the wavelength characteristic in scattering on the living tissue within a wavelength range from 400 to 2,000 nm substantially coincides with superimposed wavelength characteristics of Reyleigh scattering and Mie scattering (A. N. Bashkatov, et al., including three other authors, "Optical properties of human skin, subcutaneous and mucous tissues in the wavelength range from 400 to 2000 nm," JOURNAL OF PHYSICS D: APPLIED PHYSICS, 2005, vol. 38, p. 2543-2555, hereinafter referred to as "non-patent document 1").

**SUMMARY OF THE INVENTION**

While an endoscopic image of a living tissue is formed mainly with observation light reflected on a surface of the living tissue, the observation light may include not only the light reflected on the surface of the living tissue but also include scattered light caused in the living tissue. However, while it has been difficult to accurately determine a degree of influence of the scattered light in the captured image, the influence of the scattered light has been conventionally ignored in analysis of the spectral image. The inventor of the present invention has found a method to quantitatively evaluate the influence of the scattered light by using spectral image data, and by evaluating the observation light (i.e., an observation image) according to the method, the inventor discovered the degree of the influence of the scattered light in the observation light is greater than that having been believed so that the greater degree of influence of the scattered light has caused noise in the evaluation of the spectral property of the living tissue.

The present invention is made to solve the circumstance described above. Namely, the object of the present invention is to provide an electronic endoscope device capable of eliminating the influence of the scattered light and the like and displaying a highly contrasted image, in which the diseased portion and the healthy portion are easily recognizable.

To achieve the above described object, the electronic endoscope device according to the present invention is provided with a spectral image capturing means for capturing a spectral image in a body cavity within a predetermined wavelength range and obtaining spectral image data; a spectrum resolving means for resolving spectrum data for each of pixels contained in the spectral image data into a plurality of predetermined component spectra by performing a regression analysis; a spectrum compositing means for generating composite image data by removing at least one of the plurality of component spectra to recompose the plurality of predetermined component spectra; and a display means for displaying a screen based on the composite image data.

According to the configuration, the composite image data is generated after the component spectra acting as noise components are removed; therefore, an image, which provides higher contrast and in which the healthy portion and the diseased portion are easily identified, can be displayed.

Optionally, the plurality of component spectra includes, for example, an absorption spectrum of oxyhemoglobin, an absorption spectrum of deoxyhemoglobin, and a spectrum of a scattering coefficient. The spectrum resolving means may be configured to perform the regression analysis with the spectral data acting as an objective variable and with the absorption spectrum of oxyhemoglobin, the absorption spectrum of deoxyhemoglobin, and the spectrum of the scattering coefficient acting as explanatory variables. Optionally, the spectrum compositing means may be configured to recompose the absorption spectrum of oxyhemoglobin and the absorption spectrum of deoxyhemoglobin. In this case, it is preferable that the spectrum of the scattering coefficient includes a spectrum of a scattering coefficient in Rayleigh scattering and a spectrum of a scattering coefficient in Mie scattering. According to these configurations, by eliminating influence of the scattered light, more accurate regression coefficients of oxyhemoglobin and deoxyhemoglobin can be obtained, and purpose-specific composite image data, such as depending on concentration of oxyhemoglobin and deoxyhemoglobin, can be generated.

Optionally, the plurality of component spectra may include a spectrum indicating an offset which is specific to the electronic endoscope device. According to the configuration, the device-specific offset is removed; therefore, it is not necessary to calibrate the electronic endoscope device.

Optionally, the spectrum compositing means may be configured to obtain an average value of the recomposed component spectra and generate the composite image data with the average value acting as a pixel value. According to the configuration, the composite image data depending on the concentration of oxyhemoglobin and deoxyhemoglobin can be easily generated.

Optionally, it is preferable that the predetermined wavelength range is from 400 to 800 nm, and the spectral image includes a plurality of images captured in the wavelengths at a predetermined interval defined within a range from 1 to 10 nm.

Optionally, it is preferable that the regression analysis is a multiple regression analysis

As described above, according to the electronic endoscope of the present invention, by eliminating the influence of the scattered light and the like, it is possible to display an image, which provides higher contrast and in which the diseased portion and the healthy portion are easily identified.

## BRIEF DESCRIPTION OF THE DRAWINGS

[Fig. 1] Fig. 1 is a block diagram illustrating an electronic endoscope device according to an embodiment of the present invention.

[Fig. 2] Fig. 2 shows graphs to illustrate spectral image data of a gastric mucosa obtained by the electronic endoscope device according to the embodiment of the present invention.

[Fig. 3] Fig. 3 shows a graph illustrating an absorption property of hemoglobin.

[Fig. 4] Fig. 4 shows graphics to illustrate examples of a normal color image (an endoscopic image) and a composite spectral image.

[Fig. 5] Fig. 5 is a flowchart to illustrate an image generation process executed by an image processing unit of the electronic endoscope device according to the embodiment of the present invention.

## EMBODIMENTS FOR CARRYING OUT THE INVENTION

Hereinafter, an embodiment according to the present invention is described with reference to the accompanying drawings.

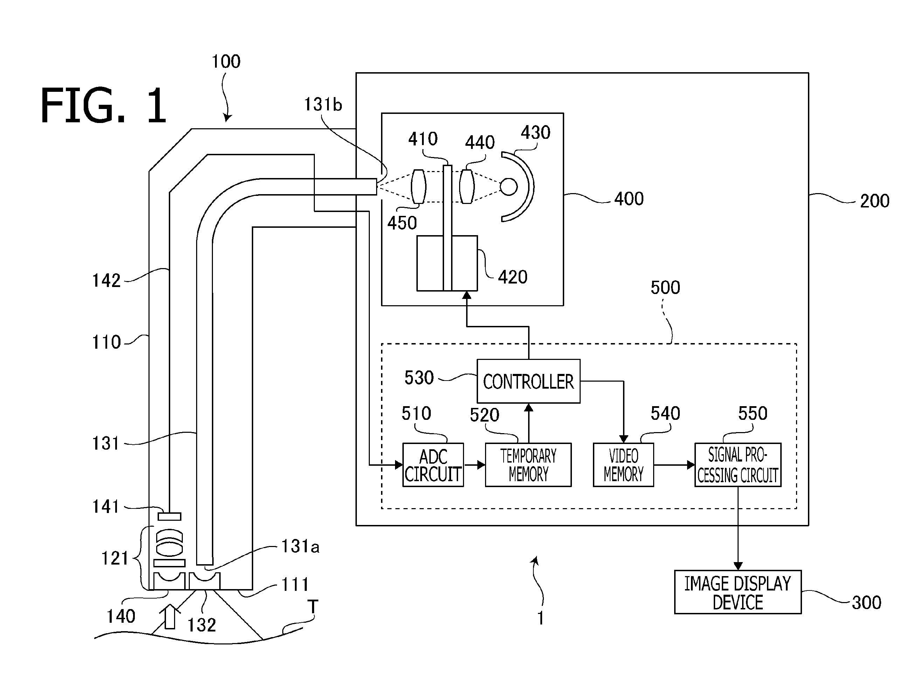

Fig. 1 is a block diagram to illustrate an electronic endoscope device 1 according to the embodiment of the invention. The electronic endoscope device 1 according to the embodiment is configured to generate a colored image (a composite spectral image), which is to be referred to by a medical doctor for diagnosing a disease of a digestive organ, such as a stomach or a rectum. The electronic endoscope device 1 includes an electronic endoscope 100, a processor 200 for the electronic endoscope, and an image display device 300. In the processor 200 for the electronic endoscope, a light source unit 400 and an image processing unit 500 are installed.

The electronic endoscope 100 includes an insertion tube 110, which is to be inserted into a body cavity. At a tip-end portion (an insertion tube tip-end portion) 111 of the insertion tube 110, disposed is an objective optical system 121. An image of a living tissue T around the insertion tube tip-end portion 111 through the objective optical system 121 is formed on a light-receiving surface of an image capturing device 141 installed in the insertion tube tip-end portion 111.

The image capturing device 141 periodically (e.g., at an interval of 1/30 seconds) outputs an image signal corresponding to the image formed on the light-receiving surface. The image signal output by the image capturing device 141 is transmitted to the image processing unit 500 in the processor 200 for the electronic endoscope via a cable 142.

The image processing unit 500 includes an AD conversion circuit 510, a temporary memory 520, a controller 530, a video memory 540, and a signal processing circuit 550. The AD conversion circuit 510 converts the image signal input from the image capturing device 141 of the electronic endoscope 100 via the cable 142 analog-to-digitally and outputs the digital image data. The digital image data output from the AD conversion circuit 510 is transmitted to the temporary memory 520 and stored therein. The controller 530 processes a piece of or a plurality of pieces of image data stored in the temporary memory 520 to generate one piece of displayable image data, and transmits the displayable image data to the video memory 540. For example, the controller 530 may produce displayable image data, such as data generated from a piece of image data, data to display a plurality of aligned images, or data to display an image obtained through image computation of a plurality of image data or a graph obtained from a result of the image computation, and store the produced displayable image data in the video memory 540. The signal processing circuit 550 converts the displayable image data stored in the video memory 540 into a video signal having a predetermined format (e.g., an NTSC format) and outputs the video signal. The video signal output from the signal processing circuit 550 is input to the image display device 300. As a result, an endoscopic image captured by the electronic endoscope 100 is displayed on the image display device 300.

In the electronic endoscope 100, a light guide 131 is installed. A tip-end portion 131a of the light guide 131 is disposed in the vicinity of the insertion tube tip-end portion 111. Meanwhile, a proximal-end portion 131b of the light guide 131 is connected to the processor 200 for the electronic endoscope. The processor 200 for the electronic endoscope includes therein the light source unit 400 (described later) having a light source 430 etc., which generates a large amount of white light, e.g., a Xenon lamp. The light generated by the light source unit 400 enters the light guide 131 through the proximal-end portion 131b. The light entering the light guide 131 through the proximal-end portion 131b is guided to the tip-end portion 131a by the light guide 131 and is emitted from the tip-end portion 131a. In the vicinity of the tip-end portion 131a of the light guide 131 in the insertion tube tip-end portion 111 of the electronic endoscope 100, a lens 132 is disposed. The light emitted from the tip-end portion 131a of the light guide 131 passes through the lens 132 and illuminates the living tissue T near the insertion tube tip-end portion 111.

As described above, the processor 200 for the electronic endoscope has both the function as a video processor, which processes the image signal output from the image capturing device 141 of the electronic endoscope 100, and the function as a light source device, which supplies illumination light for illuminating the living tissue T near the insertion tube tip-end portion 111 of the electronic endoscope 100 to the light guide 131 of the electronic endoscope 100.

In the present embodiment, the light source unit 400 in the processor 200 for the electronic endoscope includes the light source 430, a collimator lens 440, a spectral filter 410, a filter control unit 420, and a condenser lens 450. The white light emitted from the light source 430 is converted by the collimator lens 440 into a collimated beam, passes through the spectral filter 410, and then through the condenser lens 450 enters the light guide 131 from the proximal-end portion 131b. The spectral filter 410 is a disk-shaped filter, which breaks down the white light emitted from the light source 430 into light of a predetermined wavelength (i.e., selects a wavelength), and selectively filters and outputs light of a narrow band of 400 nm, 405 nm, 410 nm, ...800 nm (a bandwidth of approximately 5 nm) depending on a rotation angle thereof. The rotation angle of the spectral filter 410 is controlled by the filter control unit 420 connected to the controller 530. While the controller 530 controls the rotation angle of the spectral filter 410 via the filter control unit 420, the light of a predetermined wavelength enters the light guide 131 from the proximal-end portion 131 b, and the living tissue T near the insertion tube tip-end portion 111 is illuminated. Then, the light reflected on the living tissue T and the light scattered in the living tissue T are converged on the light-receiving surface of the image capturing device 141 to form the image as described above, and the image signal corresponding to the formed image is transmitted to the image processing unit 500 via the cable 142.

The image processing unit 500 is a device configured to obtain a plurality of spectral images at wavelengths at an interval of 5 nm from the image of the living tissue T input via the cable 142. Specifically, the image processing unit 500 obtains the spectral images of the wavelengths when the spectral filter 410 selects and outputs the light in the narrow band (a bandwidth of approximately 5 nm) having the center wavelengths of 400 nm, 405 nm, 410 nm,...800 nm respectively.

The image processing unit 500 has the function to process a plurality of spectral images generated through the spectral filter 410 and generate a colored image (a composite spectral image), as described later. Moreover, the image processing unit 500 controls the image display device 300 to display the processed composite spectral image.

As the spectral filter 410, for example, a Fabry-Perot filter or a filter employing a known spectral image capturing method, by which separated light can be obtained with use of a transmission-typed diffraction grating, may be available.

As described above, the image processing unit 500 according to the present embodiment has the function to generate a composite spectral image, which is in a high resolution and in which a healthy portion and a diseased portion can be easily recognized, by using a plurality of spectral images of different wavelengths. The function to generate the composite spectral image will be described below.

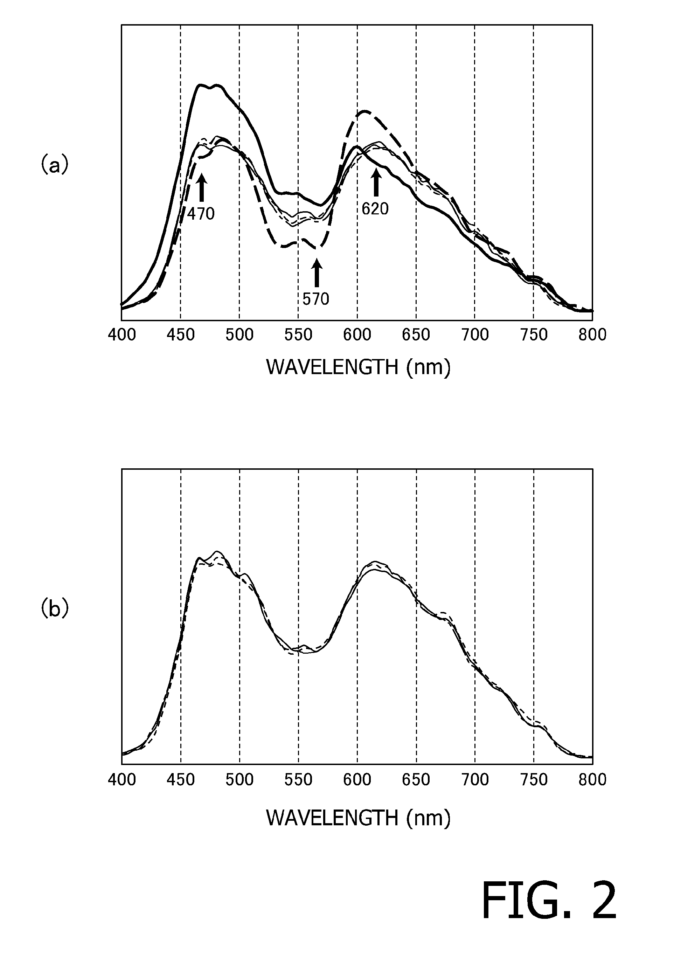

Fig. 2 shows graphs of spectral representation (i.e., representation of brightness distribution on basis of the wavelengths) of the spectral image data of a gastric mucosa obtained by the electronic endoscope device 1 according to the present embodiment. Each of the waveforms represents a spectrum of a particular pixel in a spectral image obtained by the image capturing device 141. Fig. 2(a) represents a spectrum of a pixel corresponding to a diseased portion of the gastric mucosa, and Fig. 2(b) represents a spectrum of a pixel corresponding to a healthy portion of the gastric mucosa. In this regard, a predetermined standardization process is applied to the spectrum of each pixel of the healthy portion and the diseased portion shown in Figs. 2(a) and 2(b). Specifically, while each pixel of the image capturing device 141 receives different amount of light depending on angle differences between the subject (the living tissue T) and the illumination light emitted from the tip-end portion 131a of the light guide 131 and distance differences between the insertion tube tip-end portion 111 (Fig. 1) and the living tissue T, (i.e., the image capturing device 141 is not able to receive a constant light amount over the entire light-receiving surface thereof), the spectrum representation is illustrated by correcting influences of these light amount differences. It has been proved in experiments that the spectrum of a pixel corresponding to a healthy portion and the spectrum of a pixel corresponding to a diseased portion show similar properties particularly on a higher wavelength side (i.e., they have almost no difference). Therefore, in the present embodiment, for each of spectra of pixels, brightness values of a predetermined wavelength band (e.g., the wavelength from 600 nm to 800 nm) are integrated, and the size of the entire spectrum (the brightness value at each wavelength) is corrected so that the integrated value becomes a predetermined reference value. Namely, in the present embodiment, by unifying the spectra of the pixels to match with a reference size through the standardization process, a spectrum of a pixel corresponding to a diseased portion can be precisely compared with a spectrum of a pixel corresponding to a healthy portion.

As shown in Fig. 2, the spectra of the gastric mucosa image are similar in that, regardless whether it is a healthy portion or a diseased portion, the spectrum exhibits a substantially M-shaped property having a valley (bottom) in a wavelength range from 500 nm to 590 nm, but are largely different in that the dispersion of the spectra of the pixels corresponding to the diseased portion is larger than the dispersion of the spectra of the pixels corresponding to the healthy portion, specifically in spectrum property at the wavelengths of approximately 540 nm and approximately 570 nm. This difference is widely known in the field of pathology, and it is recognized that the difference is caused by the fact that a diseased portion and a healthy portion have different component ratios of oxyhemoglobin and deoxyhemoglobin, and the light absorption property is different between oxyhemoglobin and deoxyhemoglobin. The present invention was made by focusing on the above described points, and as described later, the inventor of the present invention discovered a technique for quantitatively obtaining the component ratio of oxyhemoglobin and deoxyhemoglobin based on the difference in light absorption property between oxyhemoglobin and deoxyhemoglobin, and, by imaging the component ratio, invented a configuration for generating a composite spectral image, in which the healthy portion and the diseased portion are easily recognizable.

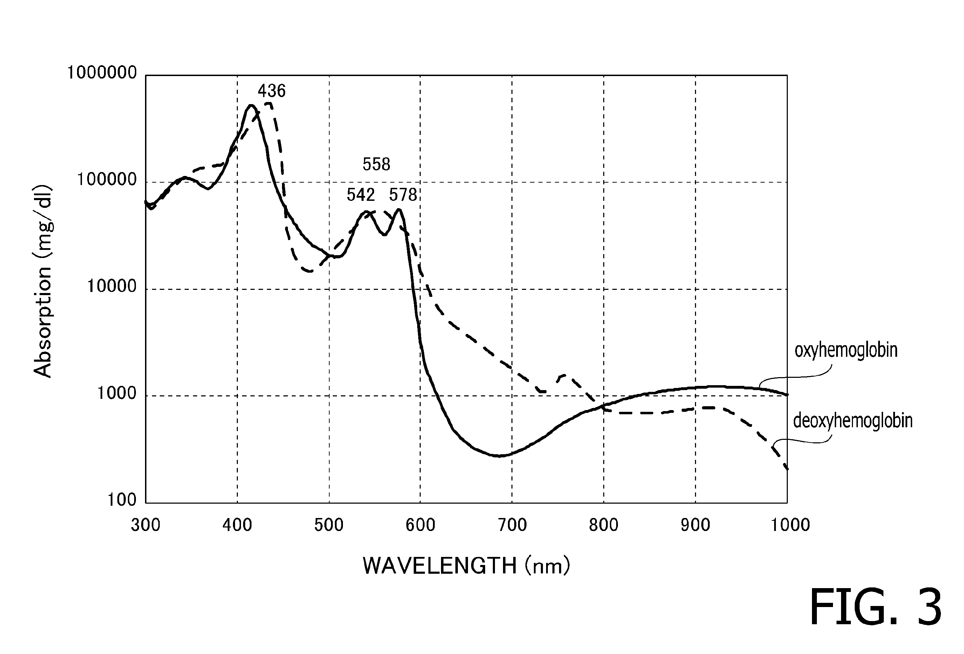

Fig. 3 is a graph representing the light absorption properties of hemoglobin, in which a solid line represents a light absorption property of oxyhemoglobin, and a dashed line represents a light absorption property of deoxyhemoglobin. In Fig. 3, the vertical axis represents the absorption (unit: mg/dl) in spectroscopy, and the horizontal axis represents the wavelength (unit: nm). As shown in Fig. 3, oxyhemoglobin and deoxyhemoglobin are common in that they absorb light with the wavelength of 500 nm to 590 nm (i.e., the absorption property increases in the wavelength range from 500 nm to 590 nm), but are different in that the property of deoxyhemoglobin has one peak at the wavelength of approximately 558 nm, whereas the property of oxyhemoglobin has two peaks at the wavelengths of approximately 542 nm and approximately 578 nm and has a bottom at the wavelength of approximately 560 nm. The absorbance of oxyhemoglobin is higher at the wavelengths of approximately 542 nm and approximately 578 nm than the absorbance of deoxyhemoglobin, and is lower at the wavelength of approximately 558 nm than the absorbance of deoxyhemoglobin. Thus, oxyhemoglobin and deoxyhemoglobin have different light absorption properties. Therefore, by performing a multiple regression analysis, with the spectral image data of the gastric mucosa shown in Fig. 2 acting as an objective variable, and with the light absorption property of oxyhemoglobin and the light absorption property of deoxyhemoglobin acting as explanatory variables, the component ratio of oxyhemoglobin and deoxyhemoglobin can be determined. However, while the spectral image data obtained by the image capturing device 141 may contain not only the light reflected on the living tissue T, it may but also contain the light scattered in the living tissue T. Further, it is assumed that the spectral image data obtained by the image capturing device 141 contain device-specific noise (errors). In order to determine the accurate component ratio of oxyhemoglobin and deoxyhemoglobin (i.e., multiple regression coefficient), it is necessary to eliminate influence of these factors. Therefore, in order to eliminate the influence of the scattering and the like, the inventor performed a multiple regression analysis with additional explanatory variables of wavelength properties by Rayleigh scattering and Mie scattering, and further with additional explanatory variables of device-specific offset which is specific to the electric endoscope device 1. As a result, the inventor discovered that the spectral image data of the gastric mucosa can be explained (i.e., fitting) substantially accurately by use of the explanatory variables, and by recomposing the image based on the obtained multiple regression coefficients of oxyhemoglobin and deoxyhemoglobin, a colored image (composite spectral image) in a high resolution, in which the healthy portion and the diseased portion can be identified, can be obtained.

A measurement model of the spectral image data is expressed in the following Expression 1.

(EXPRESSION 1)

$$X(\lambda) = A(\lambda) + S_{Mie}(\lambda) + S_{Rayleigh}(\lambda) + F$$

X represents data for a single pixel in the spectral image of the gastric mucosa (logarithmic representation), $\lambda$ represents a wavelength of light, A represents an absorption coefficient of a medium (the living tissue T), $S_{Rayleigh}$ represents a scattering coefficient of the medium in Rayleigh scattering, $S_{Mie}$ represents a scattering coefficient of the medium in Mie scattering, and F represents the device-specific offset. In this regard, the device-specific offset F is a parameter indicating a reference signal intensity for the image capturing device 141. As described in Expression 1, the spectral image data X in the present embodiment is represented in a sum of the absorption coefficient A, the scattering coefficient $S_{Rayleigh}$ in Rayleigh scattering, the scattering coefficient $S_{Mie}$ in Mie scattering, and the device-specific offset F. The absorption coefficient A is expressed as Expression 2 described below based on Beer-Lambert Law.

(EXPRESSION 2)

$$A(\lambda) = -\log_{10}\frac{I(\lambda)}{I_o(\lambda)} = \varepsilon(\lambda)Cd$$

A represents the absorption coefficient of the medium (the living tissue T), $I_0$ represents an emission intensity of light before entering the medium, I represents an intensity of light travelled in the medium for a distance of d, $\varepsilon$ represents a molar light absorption coefficient, and C represents a mol concentration. If the medium has n types of light-absorbing substances, then the absorption coefficient A is expressed in Expression 3 described below.

(EXPRESSION 3)

$$A(\lambda) = \sum_{i}^{n} \varepsilon_i(\lambda)C_i d$$

That is, when the medium has n types of light-absorbing substances, the absorption coefficient A is expressed as a sum of the absorption properties of the light-absorbing substances. Therefore, the multiple regression analysis was performed, as described below in Expression 4, with the spectral image data of the gastric mucosa shown in Fig. 2 acting as the objective variable, and with the light absorption property of oxyhemoglobin, the light absorption property of deoxyhemoglobin, the scattering coefficient of the living tissue T in Rayleigh scattering, the scattering coefficient of the living tissue T in Mie scattering, and the device-specific offset acting as the explanatory variables.

(EXPRESSION 4)

$$\begin{bmatrix} X_{400} \\ X_{405} \\ \vdots \\ X_{800} \end{bmatrix} = P1 \times \begin{bmatrix} a_{400} \\ a_{405} \\ \vdots \\ a_{800} \end{bmatrix} + P2 \times \begin{bmatrix} b_{400} \\ b_{405} \\ \vdots \\ b_{800} \end{bmatrix} + P3 \times \begin{bmatrix} c_{400} \\ c_{405} \\ \vdots \\ c_{800} \end{bmatrix} + P4 \times \begin{bmatrix} d_{400} \\ d_{405} \\ \vdots \\ d_{800} \end{bmatrix} + P5 \times \begin{bmatrix} 1 \\ 1 \\ \vdots \\ 1 \end{bmatrix}$$

X represents the data for a single pixel in the spectral image and expresses logarithmically the brightness value of the spectral image, which can be obtained by irradiating the light having the center wavelengths ranging from 400 nm to 800 nm at every 5 nm. Meanwhile, a represents the light absorption property of oxyhemoglobin at every 5 nm within the wavelengths from 400 nm to 800 nm (Fig. 3), and b represents the light absorption property of deoxyhemoglobin at every 5 nm within the wavelengths from 400 nm to 800 nm (Fig. 3). c and d represent the scattering coefficients of the medium at every 5 nm within the wavelengths from 400 nm to 800 nm in Rayleigh scattering and Mie scattering respectively. In the present embodiment, these scattering coefficients are obtained from the following expressions which are described in the non-patent document 1.

(EXPRESSION 5)

$$S_{Mie}\left(\lambda\right) = 73.7\lambda^{-0.22}$$

(EXPRESSION 6)

$$S_{Rayleigh}\left(\lambda\right) = 1.1 \times 10^{12}\lambda^{-4}$$

The last term in Expression 4 is a constant term corresponding to the device-specific offset F, and, in the present embodiment, the multiple regression coefficient P5 is equal to the device-specific offset F.

Usually, the signal intensity of the image capturing device 141 is not calibrated; therefore, it is common that an absolute value of the intensity of the video signals generated by the image capturing device 141 contains more than a small quantity of errors. Moreover, a reference level of the video signals may fluctuate depending on observation conditions (for example, ambient brightness in an observation area). If the multiple regression analysis is performed with the errors contained in the video signals maintained therein, an accurate analysis result (i.e., result with a smaller quantity of residual errors) cannot be obtained. Accordingly, in the present embodiment, by performing the multiple regression analysis in consideration of the device-specific offset as the explanatory variables, as expressed in Expression 4, the reference level is automatically and properly corrected without calibrating the signal intensity of the image capturing device 141. Thus, the multiple regression analysis with higher accuracy can be achieved.

With the multiple regression analysis (i.e., fitting) based on Expression 4, the data for the single pixel in the spectral image is resolved into spectrum of the light absorption property of oxyhemoglobin, spectrum of the light absorption property of deoxyhemoglobin, spectrum of the scattering coefficient in Rayleigh scattering, spectrum of the scattering coefficient in Mie scattering, and spectrum of the device-specific offset so that contribution rate of the spectra (component spectra) as the multiple regression coefficients P1-P5 respectively are obtained. In other words, the multiple regression coefficients P1-P5 are in fact the coefficients which indicate the component ratio of the elements (i.e., oxyhemoglobin, deoxyhemoglobin, Rayleigh scattering, Mie scattering, the device-specific offset) constituting the data for the single pixel in the spectral image. Therefore, it is possible to obtain the component ratio of oxyhemoglobin and deoxyhemoglobin in the living tissue T from the multiple regression coefficient P1 of oxyhemoglobin and the multiple regression coefficient P2 of deoxyhemoglobin obtained from the multiple regression analysis. Accordingly, thereby it is possible to substantially determine the healthy portion and the diseased portion. Meanwhile, however, in order to determine the healthy portion and the diseased portion based on the multiple regression coefficients as a type of index, it is necessary to associate the endoscopic image currently under observation with the healthy portion and the diseased portion, and to illustrate which portion in the endoscopic image is the healthy portion or the diseased portion to the operator. Therefore, in the present embodiment, by recomposing an image based on the multiple regression coefficient P1 of oxyhemoglobin and the multiple regression coefficient P2 of deoxyhemoglobin, the multiple regression coefficient P1 of oxyhemoglobin and the multiple regression coefficient P2 of deoxyhemoglobin are fed back in the endoscopic image to be shown to the operator. More specifically, by using estimate values for P1 and P2 obtained from the multiple regression analysis, composite absorption spectrum X* is generated based on Expression 7. That is, by Expression 7, only the spectrum of the light absorption property of oxyhemoglobin and the spectrum of the light absorption property of deoxyhemoglobin are recomposed.

(EXPRESSION 7)

$$\begin{bmatrix} X^*_{400} \\ X^*_{405} \\ \vdots \\ X^*_{800} \end{bmatrix} \equiv P1 \times \begin{bmatrix} a_{400} \\ a_{405} \\ \vdots \\ a_{800} \end{bmatrix} + P2 \times \begin{bmatrix} b_{400} \\ b_{405} \\ \vdots \\ b_{800} \end{bmatrix}$$

As can be seen in comparison between Expression 7 and Expression 4, in the composite absorption spectrum X*, Rayleigh scattering, Mie scattering, and the device-specific offset are regarded as noise components and are eliminated. Therefore, based on the composite absorption spectrum X*, generation of an image (composite spectral image), from which the influence of scatterings and the device-specific offset are removed, is enabled.

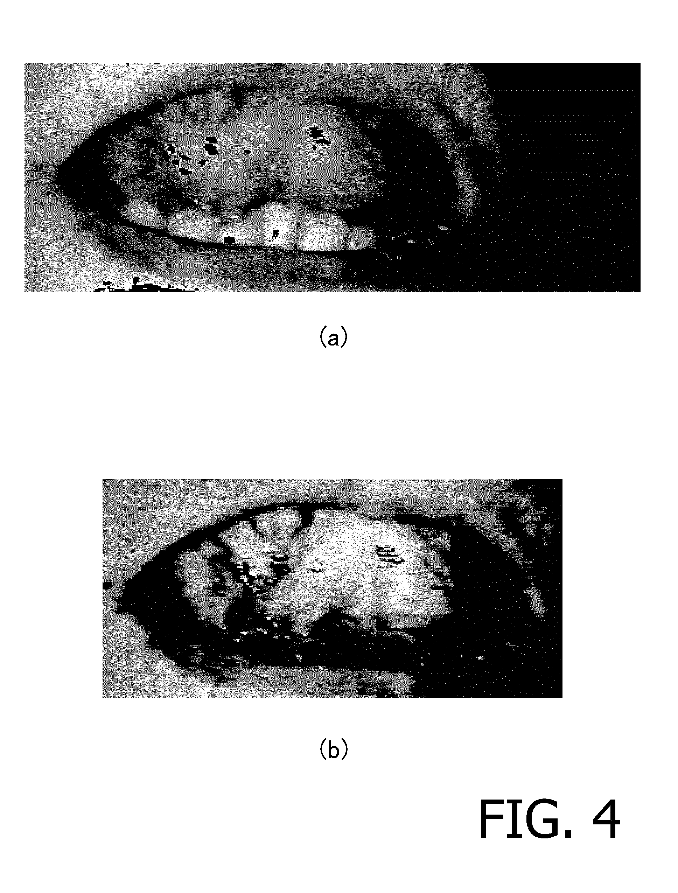

Fig. 4 shows graphics to illustrate examples of a normal colored image (an endoscopic image) and a composite spectral image. More specifically, Fig. 4(a) represents a colored image of an oral orifice, which is generated by pixel values based on spectral image spectrum captured in wavelengths at every 5 nm from 400 nm to 800 nm, and Fig. 4(b) represents a composite spectral image, which is generated by pixel values based on the composite absorption spectrum X* of Expression 7. In Figs. 4(a) and 4(b), an average value of spectrum data corresponding to each pixel is used as the pixel value to be imaged. The spectral image spectrum and the composite absorption spectrum X* both have a spectrum from 400 nm to 800 nm. Therefore, in the present embodiment, by calculating the average of the spectrum, the wavelength range is integrated so that the colored image which is equivalent to the endoscopic image captured by white light is obtained.

When the colored image in Fig. 4(a) and the composite spectral image in Fig. 4(b) are compared, it is recognized that the composite spectral image in Fig. 4(b) shows a microstructure of a sublingual tissue, which tends to contain more blood, to be brighter and clearer. Moreover, in the composite spectral image in Fig. 4(b), the scattered light is effectively eliminated; therefore, teeth containing no blood on surfaces thereof appear to be darker. In other words, by the composite spectral image of the present embodiment, the noise components such as the scattered light are eliminated, and detection accuracy of oxyhemoglobin and deoxyhemoglobin is improved. As a result, it is understood that outlines of the living tissue are emphasized, and that the healthy portion and the diseased portions are more clearly distinguished.

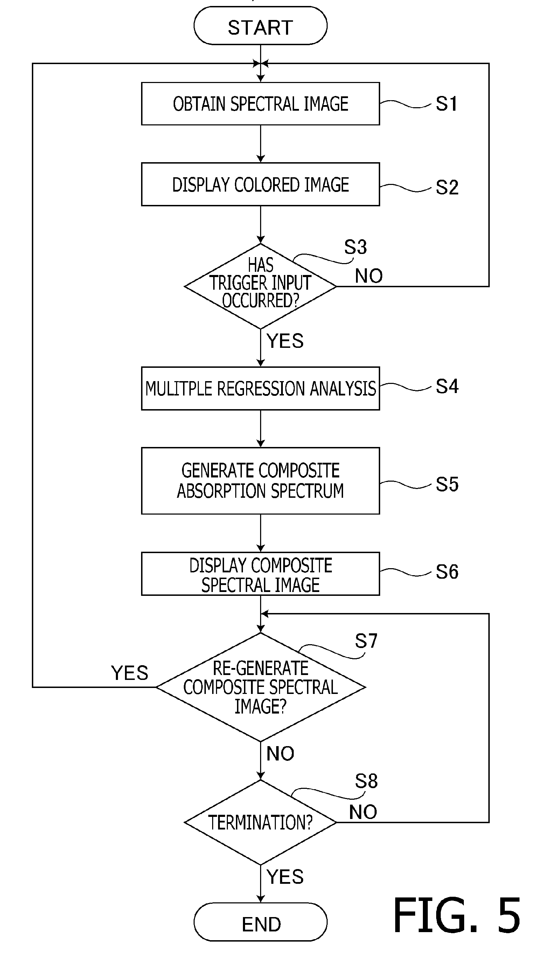

Hereafter, an image generation process executed by the image processing unit 500 according to the present embodiment is explained. Fig. 5 is a flowchart illustrating the image generation process executed by the image processing unit 500 according to the present embodiment. The image generation process is a routine to generate the above-described colored image and the composite spectral image and to display the images on the image display device 300. This routine is executed upon power-on of the electronic endoscope device 1.

When the routine is started, step S1 is processed. In step S1, the image processing unit 500 transmits a control signal to the filter control unit 400 to obtain a spectral image. When the control signal is received, the filter control unit 400 controls a rotation angle of the spectral filter 410 to sequentially select the wavelengths of light having narrow bands (a bandwidth of approximately 5nm) of 400, 405, 410, ⋯ 800 nm. The image processing unit 500 captures the spectral image obtained at each wavelength and stores it in the temporary memory 520. Then, the process proceeds to step S2.

In step S2, an average value of the spectrum data for each pixel in the spectral images obtained in step S1, and a piece of color image data is generated based on the average value acting as the pixel value. The color image data corresponds to the average brightness value of the spectrum from 400 nm to 800 nm; therefore, a colored image equivalent to the endoscopic image under normal observation (i.e., an endoscopic image obtained by white light) is generated. Then, the image processing unit 500 transmits the generated color image data to the video memory 540 and controls the image display device 300 to display the image on a left side of the screen. As a result, the image as shown in Fig. 4(a) is displayed on the image display device 300. Then, the flow proceeds to step S3.

In step S3, it is determined whether an operation unit (not shown) of the processor 200 for the electronic endoscope is operated and thereby a trigger input instructing generation of the composite spectral image occurred while the step S1 or S2 was being processed. When no trigger input occurred (S3: NO), the flow returns to S1 to obtain the spectral image again. That is, unless the trigger input occurs, the colored image obtained from the spectral image continues to be displayed on the image display device 300 in a sequential updating manner. On the other hand, when the trigger input occurred during execution of step S1 to S2 (S3: YES), the flow proceeds to step S4.

In step S4, the multiple regression analysis is performed on the spectral images obtained in step S1. More specifically, the multiple regression coefficients P1-P5 are calculated by using Expression 4 for every pixel in the spectral images obtained in step S1. Then, the flow proceeds to step S5.

In step S5, the composite absorption spectrum X* is generated by using Expression 7 for each of the multiple regression coefficients P1 and P2 calculated in step S4 for each pixel. Next, the flow proceeds to step S6.

In step S6, the composite spectral image is generated based on the composite absorption spectra X* calculated in step S5. More specifically, an average value of the composite absorption spectra X* is calculated for each pixel, and based on the average values acting as the pixel values, the composite spectral image data (composite image data) is generated. Then, the generated composite spectral image data is transmitted to the video memory 540 and is displayed on a right side of the screen on the image display device 300. As a result, the image as shown in Fig. 4(b) is displayed on the image display device 300. Thus, the image processing unit 500 arranges the composite spectral image generated from the composite absorption spectra X* and the colored image generated in step S2 on the screen of the image display device 300 side by side. Accordingly, the user (operator) of the electronic endoscope device 1 is able to perform the operation as the user compares the colored image with the composite spectral image. Then, the flow proceeds to step S7.

In step S7, the image processing unit 500 displays, on the image display device 300, a message inquiring about whether to generate again the composite spectral image and accepts an input from the operation unit (not shown) of the processor 200 for the electronic endoscope. When the user of the electronic endoscope device 1 operates the operation unit and selects re-generating of the composite spectral image (S7: YES), the flow returns to step S1. On the other hand, no re-generating of the composite spectral image is instructed for a predetermined time period (e.g., several seconds) (S7: NO), the flow proceeds to step S8.

In step S8, the image processing unit 500 displays, on the image display device 300, a message inquiring about whether to terminate displaying of the composite spectral image and accepts an input from the operation unit (not shown) of the processor 200 for the electronic endoscope. When the user of the electronic endoscope device 1 operates the operation unit and selects termination of the composite spectral image (S8: YES), the routine is terminated. On the other hand, if no displaying of the composite spectral image is instructed for a predetermined time period (e.g., several seconds) (S8: NO), the flow proceeds to step S7.

As described above, through execution of the routine shown in the flowchart of Fig. 5 by the image processing unit 500, the normal endoscopic image and the composite spectral image, by which the healthy portion and the diseased portion are effectively identified, are displayed on the image display device 300 simultaneously. By the composite spectral image being displayed, a medical doctor is able to make a diagnosis while identifying a position and a range of the diseased portion and making a comparison with a peripheral tissue.

In the present embodiment, the image processing unit 500 is in the configuration to perform the multiple regression analysis by using the entire spectral image data obtained at every 5 nm in the wavelength range from 400 to 800 nm; however, the present invention is not limited to such a configuration. For example, the wavelength range may be a narrower range including the wavelength bandwidth from 500 nm to 590 nm, which is the absorption wavelength bandwidth of oxyhemoglobin and deoxyhemoglobin, and reference values required for standardizing each pixel. For another example, a configuration to perform a multiple regression analysis by using only the spectrum image data for the wavelength bandwidth from 500 nm to 590 nm, which is the absorption wavelength bandwidth of oxyhemoglobin and deoxyhemoglobin. For another example, as long as a spectrum for a pixel corresponding to the diseased portion and a spectrum for a pixel corresponding to the healthy portion are recognizable, the spectral image data may not necessarily be obtained at the interval of 5 nm. The interval of the wavelengths to obtain the spectral image data may be, for example, selectable within a range from 1 to 10 nm.

Further, in the present embodiment, a configuration to achieve fitting by the multiple regression analysis is employed; however, another linear regression analysis, such as a multiple regression analysis of non-negative constraints and a least squares method, or an optimization method other than linear regression analyses, such as Newton's method, quasi-Newton's method, a conjugate gradient method, and a damped least squares method, may be applied as long as the fitting (optimization) is achieved based on a multivariate analysis.

## Claims

1. An electronic endoscope device, comprising:

a spectral image capturing means for capturing a spectral image in a body cavity within a predetermined wavelength range and obtaining spectral image data;
a spectrum resolving means for resolving spectrum data for each of pixels contained in the spectral image data into a plurality of predetermined component spectra by performing a regression analysis;
a spectrum compositing means for generating composite image data by removing at least one of the plurality of component spectra to recompose the plurality of predetermined component spectra; and
a display means for displaying a screen based on the composite image data.

2. The electronic endoscope device according to claim 1,
wherein the plurality of component spectra comprise an absorption spectrum of oxyhemoglobin, an absorption spectrum of deoxyhemoglobin, and a spectrum of a scattering coefficient;
wherein the spectrum resolving means performs the regression analysis with the spectral data acting as an objective variable and with the absorption spectrum of oxyhemoglobin, the absorption spectrum of deoxyhemoglobin, and the spectrum of the scattering coefficient acting as explanatory variables; and
wherein the spectrum compositing means recomposes the absorption spectrum of oxyhemoglobin and the absorption spectrum of deoxyhemoglobin.

3. The electronic endoscope device according to claim 2,
wherein the spectrum of the scattering coefficient comprises a spectrum of a scattering coefficient in Rayleigh scattering and a spectrum of a scattering coefficient in Mie scattering.

4. The electronic endoscope device according to claim 2 or claim 3,
wherein the plurality of component spectra comprise a spectrum indicating an offset which is specific to the electronic endoscope device.

5. The electronic endoscope device according to any of claims 1 through 4,
wherein the spectrum compositing means obtains an average value of the recomposed component spectra and generates the composite image data with the average value acting as a pixel value.

6. The electronic endoscope device according to any of claims 1 through 5,
wherein the predetermined wavelength range is from 400 to 800 nm; and
wherein the spectral image includes a plurality of images captured in the wavelengths at a predetermined interval defined within a range from 1 to 10 nm.

7. The electronic endoscope device according to any of claims 1 through 6,
wherein the regression analysis is a multiple regression analysis.

# FIG. 1

EP 2 850 993 A1

100

131b

410   440   430

400

200

450

420

500

CONTROLLER

530

510   520   540   550

ADC CIRCUIT → TEMPORARY MEMORY   VIDEO MEMORY → SIGNAL PRO-CESSING CIRCUIT

142

110

131

141

121

131a

111

140   132   T

1

IMAGE DISPLAY DEVICE

300

FIG. 2

FIG. 3

EP 2 850 993 A1

(a)

(b)

FIG. 4

START

OBTAIN SPECTRAL IMAGE — S1

DISPLAY COLORED IMAGE — S2

HAS TRIGGER INPUT OCCURRED? — S3 → NO

YES

MULITPLE REGRESSION ANALYSIS — S4

GENERATE COMPOSITE ABSORPTION SPECTRUM — S5

DISPLAY COMPOSITE SPECTRAL IMAGE — S6

RE-GENERATE COMPOSITE SPECTRAL IMAGE? — S7

YES

NO

TERMINATION? — S8 → NO

YES

END

FIG. 5

## INTERNATIONAL SEARCH REPORT

| | International application No. |
|---|---|
| | PCT/JP2013/061869 |

A. CLASSIFICATION OF SUBJECT MATTER
*A61B1/00*(2006.01)i, *A61B1/04*(2006.01)i, *G02B23/24*(2006.01)i, *H04N7/18*(2006.01)i

According to International Patent Classification (IPC) or to both national classification and IPC

B. FIELDS SEARCHED

Minimum documentation searched (classification system followed by classification symbols)
A61B1/00, A61B1/04, G02B23/24, H04N7/18

Documentation searched other than minimum documentation to the extent that such documents are included in the fields searched
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2011/052491 A1 (Hoya Corp.), 05 May 2011 (05.05.2011), entire text; all drawings & US 2012/0191365 A1 & CN 102595996 A | 1-7 |
| A | JP 2008-49091 A (Matsushita Electric Works, Ltd.), 06 March 2008 (06.03.2008), paragraphs [0053] to [0056] (Family: none) | 1-7 |
| P,X<br>P,A | JP 2012-143348 A (Fujifilm Corp.), 02 August 2012 (02.08.2012), paragraphs [0046] to [0078]; fig. 6 to 10 (Family: none) | 1,5-7<br>2-4 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 July, 2013 (29.07.13) | 06 August, 2013 (06.08.13) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2013/061869

C (Continuation).   DOCUMENTS CONSIDERED TO BE RELEVANT

| Category* | Citation of document, with indication, where appropriate, of the relevant passages | Relevant to claim No. |
|---|---|---|
| P,X<br>P,A | JP 2012-143349 A  (Fujifilm Corp.),<br>02 August 2012 (02.08.2012),<br>paragraphs [0049] to [0081]; fig. 6 to 10<br>& US 2012/0176486 A1    & EP 2474853 A2 | 1,5-7<br>2-4 |
| P,A | WO 2012/132571 A1  (Hoya Corp.),<br>04 October 2012 (04.10.2012),<br>entire text; all drawings<br>(Family: none) | 1-7 |

Form PCT/ISA/210 (continuation of second sheet) (July 2009)

**EP 2 850 993 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007135989 A

### Non-patent literature cited in the description

- **A. N. BASHKATOV et al.** Optical properties of human skin, subcutaneous and mucous tissues in the wavelength range from 400 to 2000 nm. *JOURNAL OF PHYSICS D: APPLIED PHYSICS,* 2005, vol. 38, 2543-2555